# EUROPEAN PATENT APPLICATION

(11) **EP 1 382 690 A1**
(43) Date of publication of application: **21.01.2004**
(21) Application number: 02077909.6
(22) Date of filing: 17.07.2002
(51) Int. Cl.: C12Q 1/37, G01N 33/68

(54) **Method to reduce false positive outcomes in prion assays**

(71) Applicant: Stichting Sanquin Bloedvoorziening, 1066 CX Amsterdam (NL)
(72) Inventor: van Oers, Josephus W. A. M., 1506 TB Zaandam (NL); van der Vorst, Teun J. K., 5702 SM Helmond (NL); Hack, Cornelis E., 1111 ND Diemen (NL); van Engelenburg, Franciscus A. C., 3555 EE Utrecht (NL)
(74) Representative: Prins, Adrianus Willem

(57) **Abstract**

This invention relates to the field of prion diseases. A method is provided to reduce false positive outcomes in a test by monitoring the activity of a proteolytic enzyme in a test sample comprising providing the test sample with a substrate and contacting the enzyme with said substrate to allow conversion of the substrate by the enzyme into a detectable product and detecting said product. Use of a method according to the invention can improve the reliability of prion tests.

## Description

This invention relates to the field of prion diseases. These diseases, also called transmissible spongiform encephalopathies (TSE), include bovine spongiform encephalopathy (BSE) or mad cow disease in cattle, scrapie in sheep, and Creutzfeldt-Jakob Disease (CJD) in man. Prion diseases are infectious diseases, generally transmitted by a conformer of the naturally present prion protein (PrP), though cases may also occur spontaneously or from a genetic predisposition. Diagnosis of TSE typically requires demonstration of this conformer in animal or human brain.

Prion diseases or TSE are a group of fatal non-inflammatory neurodegenerative disorders exemplified by BSE in cattle, scrapie in sheep or CJD in man¹. TSE are characterised by a relatively long incubation period (e.g. >10 years in human), and typically share accumulation of PrP^{Sc} in brain and in some cases also in lymphoid tissues.^{2,3} The prion protein is an endogenous protein, that also under normal conditions is expressed in the brain, and also in various other tissues. This normal cellular prion protein (PrP^{C}) and pathogenic prion (PrP^{Sc}) are different conformations of the same protein. Moreover, PrP^{C} and PrP^{Sc} differ in some physico-chemical and biochemical properties: PrP^{C} is soluble, exists in a non-aggregated form, and is sensitive to degradation by proteases, whereas PrP^{Sc} is insoluble, exist in an aggregated form, and is relatively resistant to degradation by proteases 4. It is hypothesised that a direct protein-protein contact between PrP^{Sc} and PrP^{C} is needed to generate a conformational change of PrP^{C} into PrP^{Sc}. Ultimately PrP^{Sc} forms aggregates and accumulates in the brain. At the final stage of the disease neurodegenerative changes occur in the brain, associated with severe neurological dysfunction, and ultimately death.

The PrP gene of various animal species has been cloned and sequenced.⁵ The protein is encoded within a single exon, and consists of a long N-terminal signal sequence followed by about 250 amino acid (AA) residues. The sequence of the protein is well conserved among various animal species. Though differences in the secondary structure of PrP^{Sc} and that of PrP^{C} have been identified, the structural basis for the enhanced aggregatibility and proteolytic resistance of PrP^{Sc} as compared to those of PrP^{C} is still not resolved. In addition, the molecular basis for the existence of different strains of PrP^{Sc}, i.e. prions with different incubation times, is lacking, though it is assumed that these strains likely represent PrP^{Sc} with subtle differences in conformation.⁶

Detection of prion proteins is usually achieved by several immunological methods. One method for the diagnosis of TSE is detection of PrP^{Sc} in brain, using e.g. immuno-histochemistry or Western blotting. Discrimination between the normal PrP^{C} and the pathogenic PrP^{Sc} prion protein is generally based on differences in their relative resistance to protease degradation. For the *in vitro* diagnostic methods, polyclonal and monoclonal antibodies are usually applied.^{10, 11} However, antibodies against prions described in the literature so far do not discriminate between PrP^{C} and PrP^{Sc}. Because most antibodies bind to both conformers, the specificity of commonly used TSE diagnostic methods is based on relative differences in resistance to proteinase K (PK) digestion, solubility, or degree of aggregation. Typically, a brain tissue sample to diagnose TSE is acquired from a human or animal post-mortem. Use of a method that is suitable to detect PrP^{Sc} in a more easily accessible sample, e.g. blood, is obviously preferred. There is only very incomplete data available suggesting that PrP^{Sc} is present in blood. Moreover, the high background of PrP^{C} detection in general and in blood in particular, is challenging the specificity and reliability of all current immuno-diagnostics. Thus, there is a clear need for a more sensitive and reliable test for the diagnosis of TSE.
The invention provides the insight that the milieu in a test sample often deviates from the condition for optimal protease performance. As a consequence, the outcome of a test can be less reliable. The invention thus provides a method to reduce the likelihood of the occurrence of false outcomes of a test for detecting the presence or absence of prion protein in a biological sample comprising providing the sample with a substrate and a proteolytic enzyme and contacting the enzyme with said substrate to allow conversion of the substrate by the enzyme into a detectable product allowing monitoring the activity of said enzyme in a test sample. A test sample may be a biological test sample, e.g. a biological fluid sample or a tissue homogenate. The invention provides a method to reduce false outcomes of a test by monitoring the activity of a proteolytic enzyme in a test sample. In particular, a method is provided to reduce the incidence of false positive outcomes. A test comprises one or more samples that need to be tested, for example a diagnostic prion protein test. Use of a method according to the invention is in particular advantageous for tests which rely on optimal activity of a proteolytic enzyme and wherein the efficiency of proteolysis is not analysed otherwise.

Sub-optimal protease activity in a test sample can be a particular problem when using a test set-up which depends on digestion by a protease and wherein the condition for protease activity cannot be well controlled and optimised for each individual test sample. Such situations may be encountered when the biological sample comprises e.g. a plasma sample, as plasma generally contains a high concentration of various protease inhibitors. It is described herein that protease activity is strongly reduced in the presence of plasma. In a preferred embodiment, a method according to the invention provides an internal digestion control for every individual TSE test sample. It may be used in a diagnostic TSE test comprising detection of PrP^{Sc} in easily accessible body fluid samples such as whole blood, buffy coat, serum, plasma, urine, saliva, or cerebrospinal fluid (CSF), but also in less accessible samples, like homogenates of brain or lymphoid tissue. The overall protein content in these samples is often subject to variation. Therewith, it can be difficult to estimate beforehand which quantity of proteolytic enzyme is required to ensure complete digestion of proteins in general and PrP^{C} in particular. Furthermore, such biological test samples may contain variable amounts of protease-inhibitory factors. Also, it is highly desirable to perform a quality control check for the activity of the proteolytic enzyme that is added to the test sample. Such enzymes do in general have a limited shelf life. Over time, the specific activity of an enzyme can decline progressively which often remains unnoticed by the user. Furthermore, diagnostic methods that essentially rely on the activity of enzymes should include a quality control step to check whether the test sample has received the desired quantity of active enzyme. This is particularly desirable for a diagnostic TSE test in which normal PrP^{C} and pathogenic prion protein PrP^{Sc} are discriminated based on differences in relative resistance to proteolytic degradation. It is common practice to add an enzyme to a test sample by transferring a certain volume of an enzyme-containing solution via pipetting, either manually or automatically. In those cases it can occur that a test sample receives a considerably smaller quantity of enzyme than expected. e.g. due to full or partial obstruction and / or distortion of a pipetting tip. Pipetting errors of this type are typically overlooked. The outcome of a test wherein the activity of an enzyme in each individual test sample is not controlled is therefore less reliable. In the case of a typical prion protein test, pipetting errors may result in less PK activity added to the test sample than expected. This can result in undigested PrP^{C} residues, which cannot be discriminated from PrP^{Sc} by a subsequent immunological test, giving a false positive outcome that can have far-reaching consequences.

All of the above indicate that multiple factors can interfere with protease activity and thus with the reliability of protease-based diagnostic tests. These factors include but are not limited to the presence of possible protease inhibitors in biological samples, such as plasma, and variation in the amount and / or the specific activity of the enzyme that is added to the test sample.

A method is provided for monitoring the activity of a proteolytic enzyme in a test sample wherein the enzyme activity in the sample can be affected by internal factors (e.g. protease inhibitors) and / or external factors (e.g. pipetting errors). The invention provides a control system that monitors the amount of proteolytic activity that is present in every individual test sample during digestion in tests that depend on proteolytic digestion.

The invention provides a built-in control monitoring system, giving insight in the protease activity during digestion. A method according to the invention is in particular useful for implementation in a TSE test that is based on adequate proteolytic degradation of the normal prion protein. TSE-tests often, if not always, rely on the detection of the protease-resistant core of PrP^{Sc} (PrP²⁷⁻³⁰) and may encounter the problem of incomplete digestion of normal PrP^{C}. Residual PrP^{C} may then lead to false positive results in test systems which do not monitor the size of the detected protein i.e. whether proteolysis was sub-optimal. Hence, the invention is particularly useful for diagnosis of TSE in animal and human tissues and other samples wherein the distinction between normal and pathogenic form of the prion protein is fully dependent on a reliable protease treatment. A method according to the invention can reduce the incidence of false positive data that are the result of inadequate degradation of normal prions, as exemplified herein in the detailed description.

A method is described for monitoring the activity of a proteolytic enzyme in a test sample comprising providing the test sample with a substrate and contacting the enzyme with said substrate to allow conversion of the substrate by the enzyme into a detectable product and detecting said product. The invention describes for example the use of a substrate suitable for digestion by proteinase K. This is particular useful for prion detection methods because these often depend on the selective digestion of pathogenic and normal prion protein by proteinase K or a functionally equivalent protease. For example, the invention provides a method to monitor PK activity by providing a test sample with the substrate casein-resorufin, as shown in the detailed description. Another substrate suitable for monitoring protease activity acccording to the invention is N-succinyl-Ala-Ala-Pro-Phe-p-NitroAnilide (SAAPPN).

A method according to the invention may also be used monitor the activity of another prion discriminating protease. Addition of a substrate, changing quantitatively or qualitatively in colour, enzymatic activity, fluorescence, radioactivity or molecular weight under influence of a protease, gives information on the protease activity. The substrate is for example a radiolabelled protein which upon digestion by a protease looses its radioactivity upon a certain treatment, e.g. precipitation, so that the remaining radioactivity reflects the protease activity in a sample. Even *real-time* monitoring is possible, depending on the selected protease substrate.

In a preferred embodiment, a method is provided to reduce false positive outcomes in a TSE test by for monitoring protease activity in each individual TSE test sample wherein the test comprises protein digestion by a protease and the subsequent detection of a prion protein by a prion-specific antibody. A method according to the invention is highly useful to prevent false positive outcomes in a test wherein the degree of protein degradation is not monitored by other means. For example, the method provided is especially advantageous when using an antibody that is reactive with both the protease-resistant fragment PrP^{Sc} (PrP²⁷⁻³⁰) and the incompletely digested PrP^{C}, which is often the case, and wherein the degree of protein degradation is not monitored otherwise, for example by monitoring the size of an immunoreactive protein in a Western blot analysis. Commonly used high-throughput TSE screening methods, such as an ELISA-type assay, a spot blot analysis or a dot blot analysis, do in general not entail separation of proteins according to their apparent molecular weight, e.g. by sodium dodecyl sulphate polyacrylamide gel electrophoresis (SDS-PAGE) or by size-exclusion chromatography. Also, a method is provided for the use in a TSE test comprising an antibody that reacts with digested PrP^{Sc} better than with non-digested PrP^{Sc} and both the digested and non-digested form of PrP^{C}. Such an antibody is highly specific and sensitive and can thus be used to diagnose TSE in samples that contain a low amount of PrP^{Sc} and a high amount of PrP^{C}, e.g. a blood sample. A example of such an antibody is a monoclonal antibody that can be produced by the hybridoma cell 1E4, deposited with CNCM under the accession number CNCM 1-2906. It is clear that such an antibody with a preferential reactivity with the digested form of PrP^{Sc} can only be fully exploited when an optimal protease activity is ensured. Use of a method according to the invention allows monitoring the protease activity in each individual test sample in a test that depends on proteolytic digestion so that the reliability of the outcome of such a test can be determined for each test sample.

### Figure legends

### Figure 1

### Colorimetric analysis of Proteinase K (PK) activity by casein-resorufin substrate.

Casein-resorufin was added to the sample, followed by 30 minutes of PK digestion. Subsequently, the formation of a colour relative to the PK activity was measured using a photospectrometer. As shown in the figure, a reduced PK activity in plasma samples as compared to buffer alone was observed. More details of the assay can be found in the detailed description of the invention.

### Figure 2

### Colorimetric analysis of PK activity by SAAPPN.

SAAPPN was added to the sample, followed by 30 minutes of PK digestion. Subsequently the formation of a colour relative to the PK activity was measured using a photospectrometer. As shown in the figure, a reduced PK activity in plasma samples as compared to buffer alone was observed. More details of the assay can be found in the detailed description of the invention.

### Figure 3

### Theoretical model of colorimetric analysis of PK activity bySAAPPN.

The relative differences in PK resistance of PrP^{C} and PrP^{Sc} for PK are shown graphically. At a certain PK concentration X, all PrP^{C} will be digested within a certain duration of time and most of the PrP^{Sc} present in the sample will be retained. However, when the PK activity is much lower than expected (dotted line), not all PrP^{C} will be digested, resulting in a false positive outcome in the successive immunoassay.

### Figure 4

### Implementation of the PK activity check in a diagnostic TSE ELISA test.

The test procedure is divided into separate steps. In the first step, samples are digested by PK in a 96-well PCR plate in the presence of the PK substrate N-Succinyl-Ala-Ala-Pro-Phe-p-NitroAnilide (SAAPPN) during a certain incubation period. Under optimal conditions, the amount of PK added to the samples is sufficient to completely destroy normal prion protein PrP^{C} while the infectious PrP^{Sc} protein is resistant.
In the second step, PK activity in every individual test sample is monitored in a microtitre plate reader by measuring the formation of a colour as a results of SAAPPN digestion. As shown in the Figure, a reduced PK activity is observed for well B2, indicating a sub-optimal digestion of the test sample present in well B2. In the subsequent steps, test samples are transferred to an ELISA plate and protease-resistant PrP^{Sc} remaining in the reaction mixture is detected accordingly using a prion protein-specific antibody. After completion of the ELISA test two test samples, located in well B2 and well B4, were found positive. However, based on the PK activity check results, well B2 probably gave a false positive result in the ELISA assay due to the presence of incompletely digested, immunoactive PrP^{C}. Thus, the test sample B2 has to be re-evaluated in a second test run. On the other hand, there are no indications for sub-optimal protease activity in well B4. Thus, no remaining PrP^{C} is expected in well B4 and based the ELISA result B4 has to be considered a truly positive sample.

### Detailed Description

Essential for the present invention is that detection of pathogenic prion protein in (immuno-) assays fully depends on a reliable protease treatment. E.g. discrimination between normal (PrP^{C}) and pathogenic (PrP^{Sc}) prions is based upon the relative resistance of PrP^{Sc} to cleavage by proteinase K or other proteases, subsequently remaining PrP^{Sc} fragments can be detected immunologically. However in many biological tissues and body fluids protease inhibitors can be found, possibly interfering with the proteolytic activity of the discriminating protease. Also pipetting errors may result in less PK activity than expected. This can result in undigested PrP^{C} residues, which cannot be discriminated from PrP^{Sc} by the immunological test, giving a false positive outcome that can have far-reaching consequences.

To overcome the problem of underdigestion, a marker is added to the test-sample and the activity of the prion-discrimination protease on the marker can be monitored, thus securing the presence of sufficient protease activity to digest all normal prions in the sample and preventing false positive results.

Here we describe the detection of protease activity upon cleavage by PK, but also other proteolytic enzymes can be used. Several markers can be used for this purpose, in fact every substance, changing quantitatively or qualitatively in colour, enzymatic activity, fluorescence, radioactivity or molecular weight under influence of the prion protein discriminating protease can be applied.

### Example 1

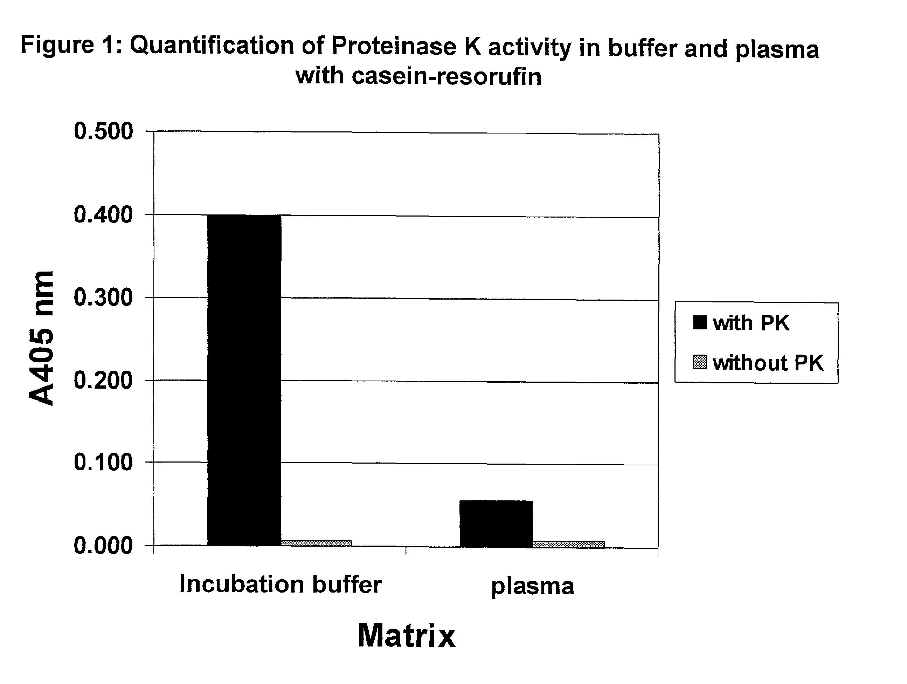

Casein-labelled resorufin is a substrate for broad series of proteases and can be used to detect protease K activity. Upon protease K treatment resorufin-labelled peptides are released from casein. Casein-resorufin can be added to each sample (brain homogenate or body fluid like serum, plasma, urine or CSF) before digestion with a protease. After 30 minutes of incubation with protease K and precipitation with trichloroacetic acid (TCA), a change in colour in the supernatant is quantified colorimetrically by measuring the absorption at 405 nanometer. If protease inhibitors were present in the sample, less protease activity will be indicated as reflected by a reduced absorption. Figure 1 shows a clear inhibitory effect of plasma on the PK activity, indicating that higher doses of PK are necessary for adequate digestion of normal prions.
By using this indicator system, information about the protease activity is available and a controlled and reliable digestion of normal prions in the sample can be expected. Subsequently only PrP^{Sc} will be detected in the following immunoassay.

### Example 2

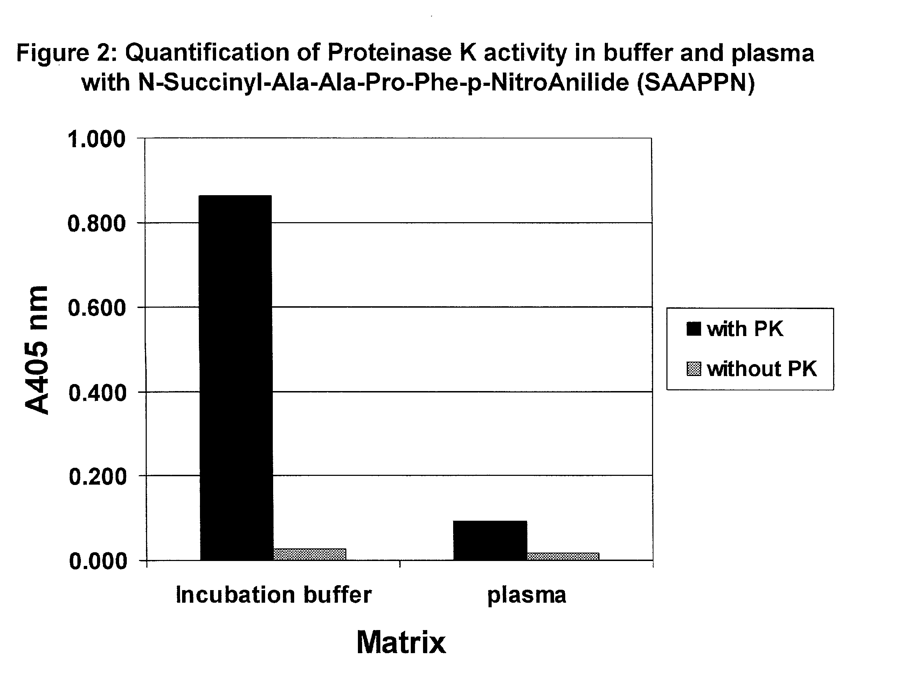

N-Succinyl-Ala-Ala-Pro-Phe-p-NitroAnilide, a chymotrypsine substrate, changes in colour accordingly to the amount of protease K activity. Figure 2 shows a clear inhibitory effect of plasma on PK activity measured by SAAPPN. This indicates that a substantially higher dose of PK is required for adequate digestion of normal prion protein in a sample that contains plasma or another substance which can inhibit PK activity.

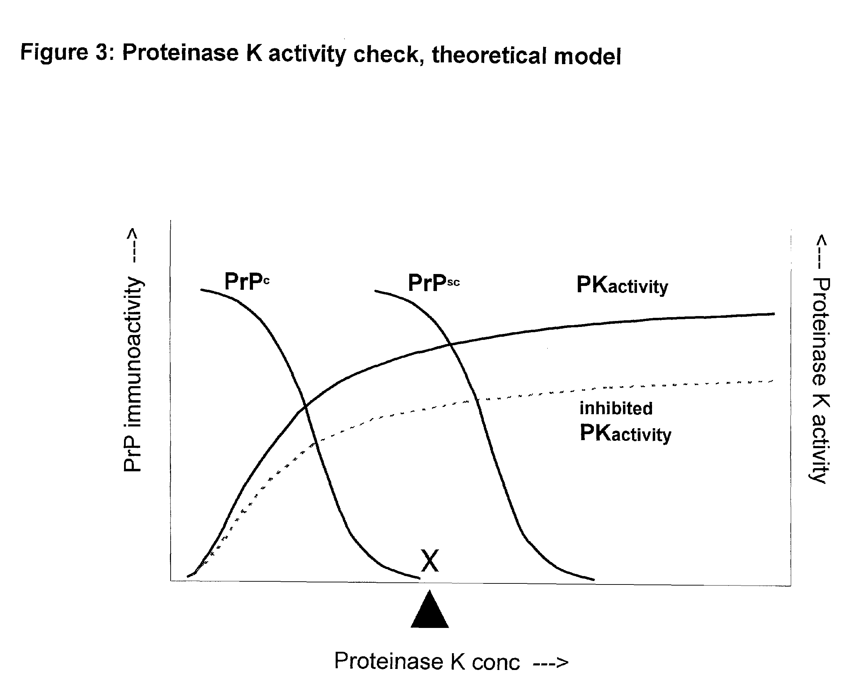

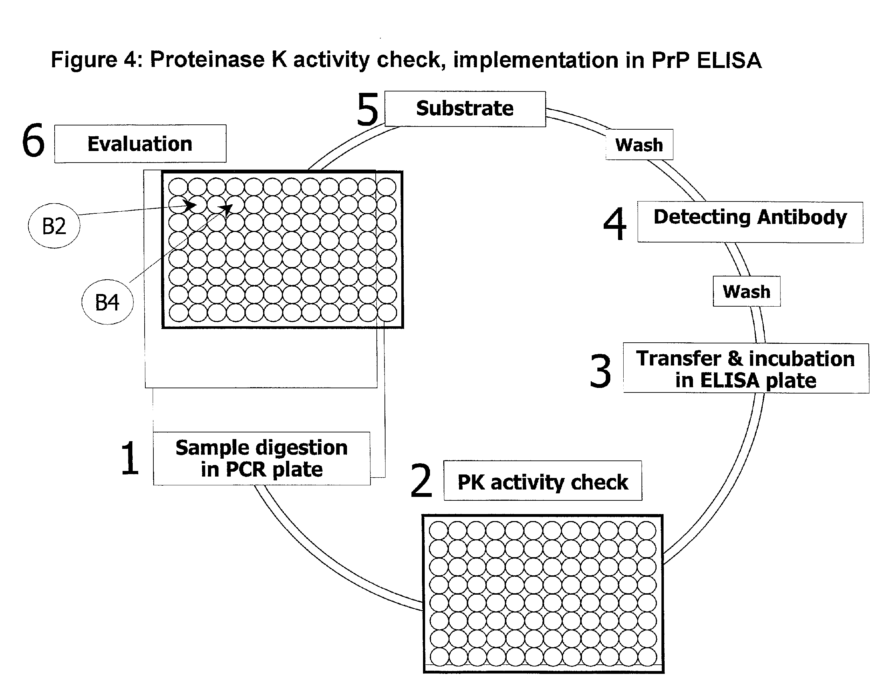

Figure 3 gives a detailed theoretical model for SAAPPN, and Figure 4 depicts an example of implementation of the SAAPPN test in any immunological TSE test.

### Other examples

Addition of an enzyme or enzyme labelled protein (e.g. Horseradish Peroxidase, Alkaline Phosphatase) to the sample before digestion. After digestion the remaining enzymatic activity will give indication of protease activity earlier.
Addition of a fluorescent protein (e.g. phyco-erythrin, aminomethylcoumarin) to the sample before digestion. After digestion the remaining fluorescence activity will give indication of protease activity earlier.

Addition of a radiolabelled protein (e.g. ¹²⁵I-IgG) to the sample before digestion. After digestion the remaining (precipitable) radioactivity will give indication of protease activity earlier.

### Methods

### Casein-resorufin detection of Proteinase K digestion activity ,

Before digestion 25 microliter of Casein-resorufin (4 mg/ml in distilled water) was added to 50 microliter sample in an Eppendorf tube and mixed. Subsequently to each tube, containing either TRIS buffer alone or 1: 5 diluted plasma sample, 25 microliter of Proteinase K (50 microgram/ml in 100 mM TRIS/HCl pH 7.5) was added . After 30 minutes of incubation at 50 °C, 50 microliter of the mixture was transferred to a separate Eppendorf tube. Subsequently 250 microliter of a 5% TCA solution was added, mixed and incubated for 10 minutes at room temperature. Finally the mixture was centrifuged for 5 minutes at 20.000 *x* g and the absorbance of the supernatant was quantified at 450 nm.

### SAAPPN detection of Proteinase K digestion activity

Before digestion 40 microliter of N-Succinyl-Ala-Ala-Pro-Phe-p-NitroAnilide (SAAPPN, 2 mM in 100 mM TRIS/HCl pH 7.5, 0.05 % SDS) was added to 40 microliter sample in a well of a 96-well format PCR plate (Biozym, The Netherlands) and mixed. Subsequently to each well, containing a mixture of SAAPPN with either TRIS buffer alone or 1:5 diluted plasma sample, 40 microliter of Proteinase K (35 microgram/ml in 100 mM TRIS/HCl, pH 7.5, 0.05 % SDS) was added. After 30 minutes of incubation at 50 °C, the absorbance of the incubation mixture was quantified at 405 nm.

### References

1 Prusiner SB, Prions (1998). PNAS 95, 13363-13383.
2 Wadsforth JDF et al., Tissue distribution of protease resistant prion protein in variant Creutzfeldt-Jakob disease using a highly sensitive immunoblotting assay (2001). Lancet 358, 171-180.
3 Schreuder BE et al., Preclinical test for prion diseases (1996). Nature 381, 563.
4 Naslavsky N, et al., Characterization of detergent-insoluble complexes containing the cellular prion protein and its scrapie isoform (1997). JBC 272, 6324-6331.
5 Wiessmann C, The ninth Datta Lecture. Molecular biology of transmissible spongiform encepholpathies (1996). FEBS Letters 389, 3-11.
6 Safar J et al., Eight prions strains have PrPSc molecules with different conformations (1998). Nature Medicine 4, 1157-1165.
7 Schmerr MJ and Jenny A, A diagnostic test for scrapie-infected sheep using a capillary electrophoresis immunoassay with fluorescent-labelled peptides (1998). Electrophoresis 19, 409-413.
8 Houston F et al. Transmission of BSE by blood transfusion in sheep (2000). Lancet 356, 999-1000
9 Shaked GM et al., A protease-resistant protein isoform is present in urine of animals and humans affected with prion diseases (2001). J.Biol Chem 276 (34), 1479-82.
10 Kascsak RJ et al., Mouse polyclonal and monoclonal antibody to scrapie-associated fibrin proteins (1987). J Virol 61, 3688-3693.
11 Laffling AJ, et al. A monoclonal antibody that enables specific immunohistological detection of prion protein in bovine spongiform encephalopathy cases (2001). Neurosci Lett. 300, 99-102.
12 Korth C et al., Prion (PrPSc)-specific epitope defined by a monoclonal antibody (1997). Nature 390, 74-77.
13 MacGregor I, Prion protein and developments in its detection (2001). Transf Med 11, 3-14.

## Claims

1. A method to reduce the likelihood of the occurrence of false outcomes of a test for detecting the presence or absence of prion protein in a biological sample comprising providing the sample with a substrate and a proteolytic enzyme and contacting the enzyme with said substrate to allow conversion of the substrate by the enzyme into a detectable product allowing monitoring the activity of said enzyme in a test sample.

2. A method according to claim 1 wherein said false outcomes comprise a false-positive outcome.

3. A method according to claims 1 or 2 wherein the outcome of said test essentially relies on the activity of said enzyme.

4. A method according to claims 1 to 3 wherein the activity of said enzyme in said test sample may be affected by an internal and/or external factor.

5. A method according to claims 1 to 4 wherein said test comprises digestion of prion protein and wherein the size of prion protein following digestion is not monitored.

6. A method according to anyone of claims 1 to 5 wherein said test comprises determining the presence of a prion protein in a sample using an antibody reactive with a prion protein.

7. A method according to claim 6 wherein said antibody reacts with both the normal and the pathogenic prion protein.

8. A method according to anyone of the preceding claims wherein said proteolytic enzyme is proteinase K or a functionally equivalent protease.

9. A method according to anyone of the preceding claims wherein said substrate is suitable for digestion by proteinase K.

10. A method according to anyone of claims 1 to 9 wherein said substrate is a chymotrypsin substrate.

11. A method according to anyone of claims 1 to 9 wherein said substrate is casein-resorufin.

12. A method according to anyone of claims 1 to 9 wherein said substrate is N-succinyl-Ala-Ala-Pro-Phe-p-NitroAnilide (SAAPPN).

13. A method according to anyone of claims 1 to 9 wherein said substrate has an enzymatic label.

14. A method according to anyone of claims 1 to 9 wherein said substrate is a fluorescent protein.

15. A method according to anyone of claims 1 to 9 wherein said substrate is a radiolabelled protein.

16. Use of a method according to anyone of the preceding claims to monitor protease activity in each individual test sample in a test which essentially depends on proteolytic digestion so that the reliability of the outcome of such a test can be determined for each test sample.
